Europäisches Patentamt

European Patent Office

Office européen des brevets

(11) Veröffentlichungsnummer: **0 222 236**
**B1**

(12) **EUROPÄISCHE PATENTSCHRIFT**

(45) Veröffentlichungstag der Patentschrift:
18.10.89

(51) Int. Cl.⁴: **A61F 2/36**

(21) Anmeldenummer: **86114839.3**

(22) Anmeldetag: **25.10.86**

(54) Geradschaft für eine Femurkopfprothese.

(30) Priorität: **14.11.85 CH 4878/85**

(43) Veröffentlichungstag der Anmeldung:
**20.05.87 Patentblatt 87/21**

(45) Bekanntmachung des Hinweises auf die Patenterteilung:
**18.10.89 Patentblatt 89/42**

(84) Benannte Vertragsstaaten:
**AT DE FR GB IT**

(56) Entgegenhaltungen:
**EP-A- 0 050 533**
**EP-A- 0 145 939**
**EP-A- 0 159 462**
**DE-A- 2 607 315**
**DE-A- 3 505 997**

(73) Patentinhaber: **GEBRÜDER SULZER
AKTIENGESELLSCHAFT, Zürcherstrasse 9,
CH-8401 Winterthur(CH)**

(72) Erfinder: **Stühmer, Karl-Gerhart, Dr.-med., St.
Elisabethen-Krankenhaus, D-7980 Ravensburg(DE)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf 1(DE)**

## Beschreibung

Die Erfindung betrifft einen Geradschaft für eine Femurkopfprothese, wobei der Geradschaft in seinem distalen Bereich kreiszylindrisch ausgebildet ist, wobei der distale Kreiszylinder in einen proximalen Teil mit etwa rechteckförmigen Querschnitt übergeht, wobei der proximale Teil sich entlang der Schafthöhe nach lateral und medial stetig erweitert und mit parallel zu seiner Längsachse verlaufenden Rippen versehen.

Ein Schaft der vorstehend genannten Art ist aus der CH-A 560 042 bekannt; er ist für eine zementfreie Verankerung bestimmt und wird in seinem distalen Bereich fixiert, der zu diesem Zweck mit Vertiefungen und Rippen in Umfangsrichtung ausgestattet ist. Weitere parallel zur Längsachse verlaufende Nuten und Rippen sind im im wesentlichen einen rechteckigen Querschnitt aufweisenden proximalen Teil vorgesehen.

In den letzten Jahren besteht die Tendenz, den Schaft einer Hüftgelenkprothese möglichst nahe an seinem proximalen Ende zu fixieren und ihn im distalen Bereich in Richtung seiner Längsachse lediglich in einem Gleitsitz zu führen (siehe z.B. die unter Art. 54(3) EPÜ fallende EP-A 0 187 903). Dabei hat sich herausgestellt, dass bei zementfreier Implantation im proximalen Bereich eine Struktur der Schaftoberfläche gewählt werden muss, die möglichst tief und grossflächig in das spongiöse Gewebe eindringt. Da der Schaftkern wegen der Festigkeit eine gewisse Mindestdicke haben muss, ergeben sich aus anatomischen Gründen – wegen der Querschnittsform des Femurknochens und der Verteilung der Spongiosa in seinem proximalen Bereich unterhalb des Schenkelhalses – Schwierigkeiten, bei den bisherigen Schaftformen relativ grossflächige Rippen in genügender Zahl an dem Schaftkern unterzubringen, ohne die Gefahr einer Sprengung der kortikalen Knochenschalen in Kauf nehmen zu müssen.

Aufgabe der Erfindung ist es, diese Schwierigkeiten zu überwinden und einen Prothesenschaft der eingangs genannten Art zu schaffen, bei dem trotz grossflächiger, möglichst die ganze Spongiosa durchsetzender Rippen keine Gefahr besteht, dass die kortikale Knochenschale beim Einschlagen des Schaftes in den Femur gesprengt wird. Diese Aufgabe wird mit der Erfindung dadurch gelöst, dass der Schaftkern an seinem proximalen Ende derart gebogen ist, dass der Querschnitt des Schaftkerns eine bananenähnliche Form erhält, wobei der Bogen nach distal allmählich in eine Gerade übergeht, und dass ferner die Mehrzahl der Rippen im konkaven Bereich des Bogens eine grössere Höhe als auf der konvexen Seite hat, so dass die umhüllende Aussenbegrenzung des Schaftes im Querschnitt linsenförmig ist.

Die auf diese Weise erzeugte unsymmetrische Form des Schaftes bezüglich seiner Mittelebene bedingt, dass für die linke und rechte Körperseite verschiedene Schäfte vorzusehen sind. Die bananenähnliche Krümmung im proximalen Bereich und die linsenförmige Aussenform des Schaftes tragen der Anatomie des Femurknochens unterhalb des Schenkelhalsbereichs Rechnung, in dem sich ein relativ flacher ovaler Querschnitt für die Spongiosa ergibt, die beidseitig der Mittelebene ungleichmässig verteilt ist.

Mit der neuen Form des Schaftes füllt die Rippenstruktur diesen Querschnitt in optimaler Weise aus.

Die Antetorsion des natürlichen Schenkelhalses lässt sich berücksichtigen, wenn die Prothesenhalsachse mit der Längs mittelebene des Schaftes zur konkaven Seite des Kernbogens hin einen Winkel zwischen 5° und 15° bildet. Weiterhin ist es zweckmässig, als zusätzliche Rotationssicherung lateral am Schaftkern einen Trochanterflügel anzuordnen, der in Richtung der Mittelebene des nicht gebogenen Schaftkernes verläuft. Das Einschlagen der Rippenstruktur in die Spongiosa und damit die Arbeit des Operateurs lassen sich erleichtern, wenn der Trochanterflügel und die Rippen durch scharfe Messerprofile gebildet sind.

Das eingangs erwähnte Gleiten des Schaftes im distalen Bereich – z.B. wenn die Prothese sich im Laufe der Zeit "setzt " – kann erleichtert werden, wenn im distalen Kreiszylinder eine zentrale axiale Längsbohrung vorgesehen ist, deren Begrenzung durch kreuzförmig angeordnete Einschnitte in Sektoren unterteilt ist und/oder wenn die Oberfläche im Bereich des Kreiszylinders poliert, im proximalen Teil jedoch strukturiert ist.

Als Materialien für den neuen Schaft können alle in der Implantattechnik verwendeten Metalle, vorzugsweise Titan oder eine Titanlegierung, dienen.

Im folgenden wird die Erfindung anhand eines Ausführungsbeispiels näher erläutert.

Fig. 1 ist eine Seitenansicht des neuen Schaftes;

In Fig. 2a–f. sind über die Höhe verteilte Schnitte A–A bis F–F des Schaftes nach Fig. 1 wiedergegeben;

Fig. 3 schliesslich zeigt in einer Aufsicht einen für die rechte Körperseite bestimmten Schaft nach Fig. 1 implantiert in einen Femurknochen.

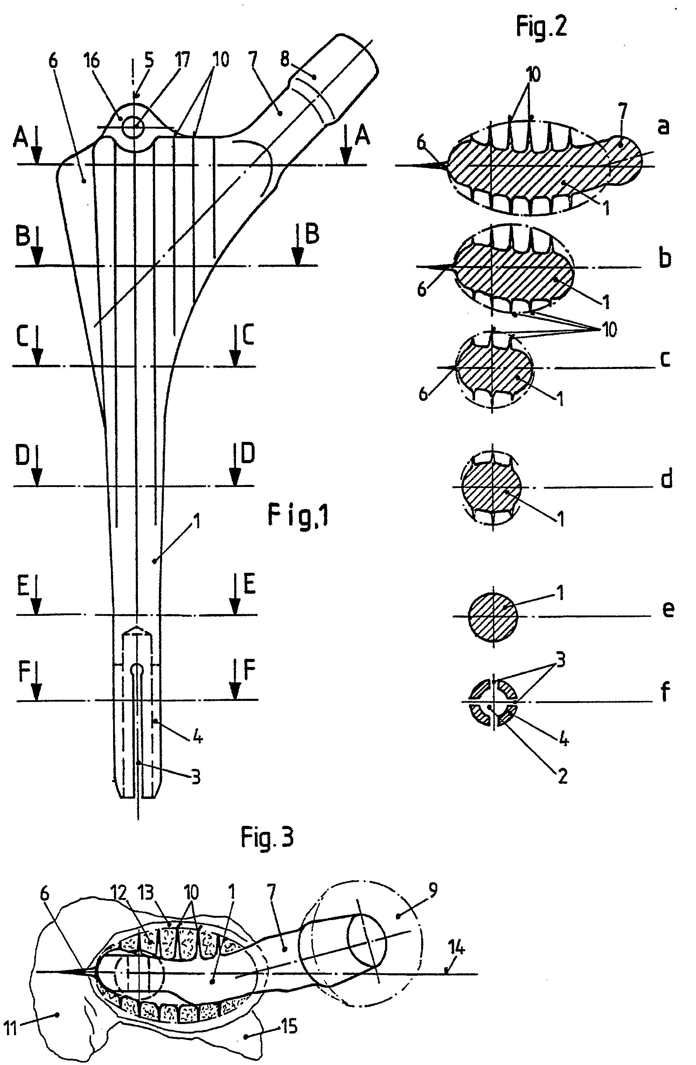

Im distalen Bereich – etwa bis zur Höhe des Schnittes E–E – ist der neue Schaft 1 kreiszylindrisch ausgebildet; seine Oberfläche in diesem Bereich ist glattpoliert, um seine Gleiteigenschaften zu verbessern. Vom Ende her durchsetzt eine zentrale axiale Bohrung 2 (Fig. 2f) den ganzen distalen Bereich. Zusätzlich sind in die verbleibende Wand 4 Schlitze 3 eingeschnitten; Bohrung 2 und Schlitze 4 verleihen den Segmenten der Wand 4 eine Beweglichkeit, die dem Schaft ein Nachgeben gegenüber Unebenheiten im ausgeräumten Markkanal erlaubt.

Im Anschluss an den zylindrischen Teil geht der Schaft in einen proximalen Teil über, dessen Querschnitt im wesentlichen ein Rechteck bildet. Nach lateral und medial erweitert sich dieser Teil zunächst stetig; er geht etwa auf halber Schafthöhe – gemessen entlang der Längsachse 5 – lateral in einen Trochanterflügel 6 und medial in einen Bogen über, der seinerseits im Prothesenhals 7 endet. Dieser trägt einen konischen Zapfen 8 für den in Fig. 1 nicht gezeigten Gelenkkopf 9 (Fig. 3).

Die von den Länggsseiten der Rechteckquerschnitte des Schaftkernes gebildeten Schaftoberflächen sind mit zur Längsachse 5 parallelen Rippen 10 bedeckt, die – ebenso wie der Trochanterflügel 6 – als scharfe Messerprofile ausgebildet sind. Den proximalen Abschluss des geraden Schaftteiles bildet eine horizontale Schulter, die vom Trochanterflügel 6 zum Prothesenhals 7 verläuft. Aus ihr ragt ein Vorsprung 16 hervor, in dem eine Bohrung 17 vorgesehen ist; diese dient als Angriffspunkt für ein Ausziehinstrument bei eventuellen Reoperationen.

Oberhalb etwa des Schnittes C–C ist der Schaftkern als Ganzes gebogen, so dass der Querschnitt eine bananenähnliche Form erhält. Die Krümmung des Bogens nimmt dabei zum proximalen Ende hin zu. Wie die Schnitte A–A und B–B zeigen, haben im Bereich des Bogens die Rippen 10 auf der konvexen und auf der konkaven Bogenfläche unterschiedliche, zusätzlich von der Mitte nach aussen abnehmende Höhen, so dass die umhüllende Aussenform des Gesamtschaftes 1 einer relativ starken Konvexlinse ähnelt.

Diese Linsenform passt sich, wie Fig. 3 erkennen lässt, optimal in den ovalen Querschnitt des Femurknochens 11 ein, wobei die schneidenden Messerrippen 10 und der Trochanterflügel 6 in die Spongiosa 12 eindringen. Durch die Bananenform des Schaftkerns wird dabei ermöglicht, dass die Rippen 10 auf beiden Seiten bis nahe an die harte kortikale Schale 13 heranreichen. Neben einer festen Fixierung wird so eine optimale Rotationssicherung erreicht.

Der Prothesenhals 7 und damit der auf ihm sitzende Gelenkkopf 9 sind aus der Mittelebene 14 der Prothese – wie man aus der Lage des leicht nach posterior weisenden kleinen Trochanter 15 entnehmen kann – um einen Winkel nach anterior herausgedreht, der einen Wert zwischen 5° und 15° annehmen kann. Durch diese zusätzliche Massnahme wird die Lage des Gelenkkopfes 9 optimal an die Stellung des natürlichen Femurkopfes angepasst, der aufgrund der Antetorsion und der Spiralform des Femurknochens ebenfalls nach anterior "verdreht" ist.

## Patentansprüche

1. Geradschaft (1) für eine Femurkopfprothese, wobei der Geradschaft in seinem distalen Bereich kreiszylindrisch ausgebildet ist, wobei der distale Kreisylinder (4) in einen proximalen Teil mit etwa rechteckförmigem Querschnitt übergeht, wobei der proximale Teil sich entlang der Schafthöhe nach lateral und medial stetig erweitert und mit parallel zu seiner Längsachse (5) verlaufenden Rippen (10) versehen ist, dadurch gekennzeichnet, dass der Schaftkern an seinem proximalen Ende derart gebogen ist, dass der Querschnitt des Schaftkerns eine bananenähnliche Form erhält, wobei der Bogen nach distal allmählich in eine Gerade übergeht, und dass ferner die Mehrzahl der Rippen (10) im konkaven Bereich des Bogens eine grössere Höhe als auf der konvexen Seite hat, so dass die umhüllende Aussenbegrenzung des Schaftes (1) im Querschnitt linsenförmig ist.

2. Geradschaft nach Anspruch I, dadurch gekennzeichnet, dass die Prothesenhalsachse (7) mit der Längsmittelebene (I4) des Schaftes (I) zur konkaven Seite des Kernbogens einen Winkel zwischen 5° und 15° bildet.

3. Geradschaft nach Anspruch I oder 2, dadurch gekennzeichnet, dass lateral am Schaftkern ein Trochanterflügel (6) angeordnet ist, der in Richtung der Mittelebene (I4) des nicht gebogenen Schaftkernes verläuft.

4. Geradschaft nach einem der Ansprüche I - 3, dadurch gekennzeichnet, dass der Trochanterflügel (6) und die Rippen (I0) durch scharfe Messerprofile gebildet sind.

5. Geradschaft nach einem der Ansprüche I - 4, dadurch gekennzeichnet, dass im distalen Kreiszylinder (4) eine zentrale axiale Längsbohrung (2) vorgesehen ist, deren Begrenzung durch kreuzförmig angeordnete Einschnitte (3) in Sektoren unterteilt ist.

6. Geradschaft nach einem der Ansprüche I - 5, dadurch gekennzeichnet, dass die Oberfläche im Bereich des Kreiszylinders (4) poliert, im proximalen Teil jedoch strukturiert ist.

## Claims

1. A straight stem (1) for a prosthetic femur head, the stem being cylindrical in its distal zone, the distal cylinder (4) mering into a proximal part of substantially rectangular cross-section, the proximal part widening continuously along stem height in the lateral and medial directions and having ribs (10) which extend parallel to its longitudinal axis (5), characterized in that the stem core is so curved at its proximal end that the cross-section of the stem core in shape resembles a banana, the curve gradually merging distally into a straight line, and the number of ribs (10) in the concave zone of the curve are of greater height than on the convex side so that the envelope outer boundary of the stem (1) is in cross-section lenticular.

2. A stem according to claim 1, characterized in that the axis (7) of the neck of the prosthesis forms an angle between 5° and 15° with the longitudinal centre-plane (14) of the stem (1) towards the concave side of the core curve.

3. A stem according to claim 1 or 2, characterized in that a trochanter lobe (6) is disposed laterally on the stem core and extends in the direction of the centre plane (14) of the uncurved stem core.

4. A stem according to any of claims 1–3, characterized in that the trochanter lobe (6) and the ribs (10) are embodied by sharp knife profiles.

5. A stem according to any of claims 1–4, characterized in that the distal cylinder (4) is formed with a central axial longitudinal bore (2) whose boundary is subdivided into sectors by cruciform incisions (3).

6. A stem according to any of claims 1–5, characterized in that the surface is polished near the cylinder (4) but structured in the proximal part.

## Revendications

1. Tige rectiligne (1) pour une prothèse de tête fémorale, cette tige rectiligne étant de réalisation cylindrique droite dans sa zone distale, le cylindre droit distal (4) se prolongeant par une partie proximale de section sensiblement rectangulaire, la partie proximale s'évasant en permanence le long de la hauteur de la tige, vers les zones latérale et médiale, et étant pourvue de nervures (10) s'étendant parallèlement à son axe longitudinal (5), caractérisée par le fait que l'âme de la tige présente, à son extrémité proximale, un cintrage propre à conférer, à la section de cette âme de la tige, une forme similaire à celle d'une banane, le cintre se prolongeant progressivement par une ligne droite, vers la zone distale; et par le fait que, en outre, la plupart des nervures (10) possèdent, dans la région concave du cintre, une plus grande hauteur que du côté convexe, de sorte que la délimitation extérieure ceinturant la tige (1) est de section lentiforme.

2. Tige rectiligne selon la revendication 1, caractérisée par le fait que l'axe (7) du col de la prothèse forme avec le plan médian longitudinal (14) de la tige (1), en direction du côté concave du cintre de l'âme, un angle compris entre 5° et 15°.

3. Tige rectiligne selon la revendication 1 ou 2, caractérisée par le fait qu'une aile trochantérienne (6), disposée sur l'âme de la tige dans la zone latérale, s'étend dans la direction du plan médian (14) de l'âme noncintrée de la tige.

4. Tige rectiligne selon l'une des revendications 1–3, caractérisée par le fait que l'aile trochantérienne (6) et les nervures (10) sont formées par des profils de lames effilés.

5. Tige rectiligne selon l'une des revendications 1–4, caractérisée par le fait qu'il est prévu, dans le cylindre droit distal (4), un perçage longitudinal (2), axial et central, dont la délimitation est scindée en des secteurs par des entailles (3) ménagées en croix.

6. Tige rectiligne selon l'une des revendications 1–5, caractérisée par le fait que la surface est polie au voisinage du cylindre droit (4), mais est cependant structurée dans la partie proximale.

Fig.1

Fig.2

Fig.3